# EUROPEAN PATENT APPLICATION

(11) **EP 4 631 868 A2**
(43) Date of publication of application: **15.10.2025**
(21) Application number: 25157018.0
(22) Date of filing: 11.02.2025
(51) Int. Cl.: B65B 9/067, A61F 13/551, B29C 65/00, B65B 25/14, B65B 51/16, B65B 61/06, B65B 63/04

(54) **A METHOD AND A MACHINE FOR PACKAGING ABSORBENT SANITARY ARTICLES**

(30) Priority: 26.02.2024 IT 202400004048
(71) Applicant: Fameccanica.Data S.p.A., 66020 San Giovanni Teatino (CH) (IT)
(72) Inventor: MANCINI, Osvaldo, 66020 San Giovanni Teatino (Chieti) (IT); GUALTIERI, Diego, 66020 San Giovanni Teatino (Chieti) (IT); CENTORAME, Oscar, 66020 San Giovanni Teatino (Chieti) (IT); BONELLI, Guido, 66020 San Giovanni Teatino (Chieti) (IT)
(74) Representative: Buzzi, Notaro & Antonielli d'Oulx S.p.A.

(57) **Abstract**

A method for packaging absorbent sanitary articles enclosed in respective paper material wrappers. A machine for producing absorbent sanitary articles enclosed in respective paper material wrappers comprising a packaging apparatus.

## Description

### Field of the Invention

The present invention relates in general to the packaging of absorbent sanitary articles, particularly folded absorbent sanitary articles whose thickness is smaller compared to the other dimensions of the folded article.

More specifically, the invention relates to a method for packaging absorbent sanitary articles in paper material wrappers.

The invention has been developed in particular for packaging feminine hygiene products such as pantyliners, light incontinence pads, sanitary liners, or similar items.

In the following description, reference will be made to this specific field of application without, however, losing generality.

According to another aspect, the invention also relates to a machine for producing absorbent sanitary articles enclosed in respective wrappers.

### Description of the Prior Art

Absorbent sanitary articles with a thin shape, such as feminine hygiene products, are often individually packaged in respective wrappers commonly called pouches.

The individual packaging of sanitary articles can be achieved by enclosing each absorbent sanitary article between two flexible sheets placed over one another, sandwiching the absorbent sanitary article, and joined together at their edges using adhesive.

Solutions are also known in which each absorbent sanitary article is enclosed in a wrapper formed by a single sheet of flexible material, folded to form two or three flaps joined together at their edges using adhesive.

The wrappers can be made of plastic sheets, such as polyethylene, or paper material.

Manufacturers and users of packaging materials are increasingly opting for paper material as a substitute for plastic material to reduce environmental impact.

However, in the field of packaging sanitary articles, the environmental sustainability benefits derived from using paper material instead of plastic sheets are partially negated by the fact that paper material wrappers are sealed with synthetic adhesives that compromise the biodegradability of the areas where the adhesive is applied.

From document IT202022000000080 by the same applicant, a consumer product is known that includes an absorbent sanitary article enclosed in a paper material wrapper sealed with a biodegradable adhesive.

Using a biodegradable adhesive maintains the biodegradability of the packaging. However, the solution known from this document requires the application of adhesive strips along the edges of the paper material sheets, which leads to several problems on the packaging line.

### Object and summary of the invention

The object of the present invention is to provide a method for packaging absorbent sanitary articles that overcomes the problems of the prior art.

According to the present invention, this object is achieved by a method having the features forming the subject of claim 1.

According to another aspect, the invention relates to a machine for producing absorbent sanitary articles enclosed in respective wrappers according to claim 10.

Preferred embodiments are the subject of the dependent claims.

### Brief description of the drawings

The present invention will now be described in detail with reference to the accompanying drawings, provided solely by way of non-limiting example, in which:
- Figures 1-6, 7-11, 12-16, and 17-20 are schematic plan views respectively illustrating a first, second, third, and fourth embodiment of a method for packaging absorbent sanitary articles according to the present invention,
- Figure 21 is a schematic side view illustrating a rotating embossing unit,
- Figures 22A, 22B, and 22C are enlarged details of the part indicated by arrow XXII in Figure 21,
- Figure 23 is an enlarged detail illustrating the embossing area of two layers of paper material placed over one another,
- Figure 24 is a perspective view of a detail of an embodiment of an embossing unit,

Figures 25-28 are perspective views illustrating four variants of a method for packaging absorbent sanitary articles that do not fall within the claims.

### Detailed description

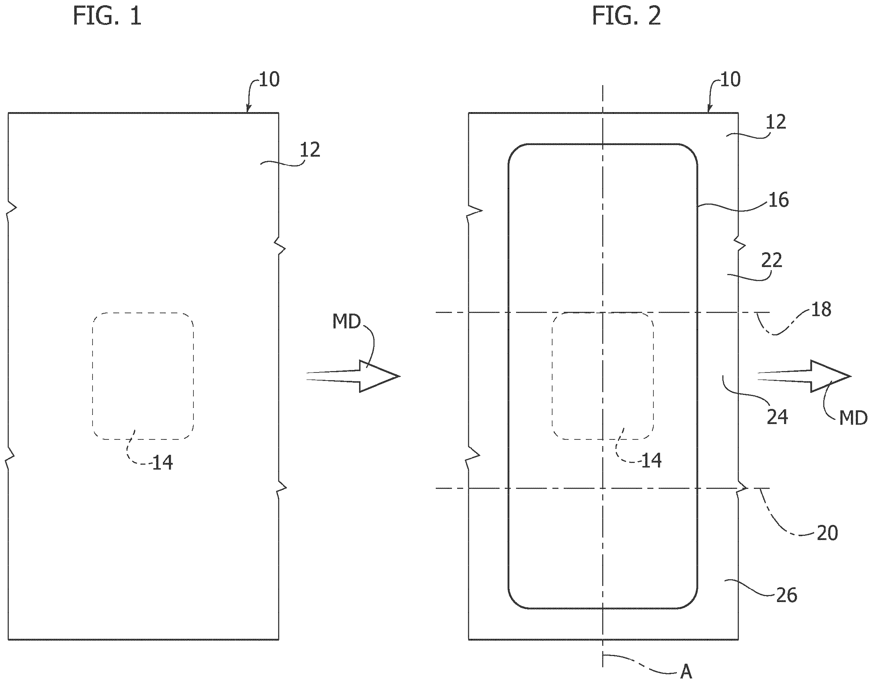

Figures 1-20 illustrate various embodiments of a method for packaging absorbent sanitary articles.

The absorbent sanitary articles can include feminine hygiene products such as pantyliners, light incontinence pads, sanitary liners, or similar items.

The method involves advancing in a machine direction MD a sheet of paper material 10 having a surface covered with a silicone layer 12.

The sheet of paper material 10 can be made of biodegradable kraft paper. The paper material 10 can be compostable as well as biodegradable. The paper material may have a basis weight between 25-50 g/m². The silicone layer 12 can extend uniformly across the entire surface of the sheet of paper material 10 and may have a basis weight between 1-3 g/m².

The method includes applying adhesive on the silicone layer 12 to form an adhesive area 14 on the sheet of paper material 10.

Then, the method provides for placing an absorbent sanitary article 16 onto the adhesive area 14 of the sheet of paper material 10. At this stage, the absorbent sanitary article 16 is in a flat position and is parallel to the sheet of paper material 10.

Thanks to the silicone layer 12, when the absorbent sanitary article 16 is removed from the sheet of paper material 10 at the time of use, the adhesive detaches from the silicone layer 12 and transfers onto the absorbent sanitary article 16. During use, the adhesive serves to secure the absorbent sanitary article 16 to the inner surface of an undergarment.

In possible embodiments, the absorbent sanitary article 16 can be secured to the sheet of paper material without using adhesives, for example, through thermal welding, ultrasonic welding, or embossing.

Next, the method involves jointly folding the sheet of paper material 10 and the absorbent sanitary article 16 along at least one fold line 18, 20 to form at least two overlapping flaps of paper material 22, 24, 26.

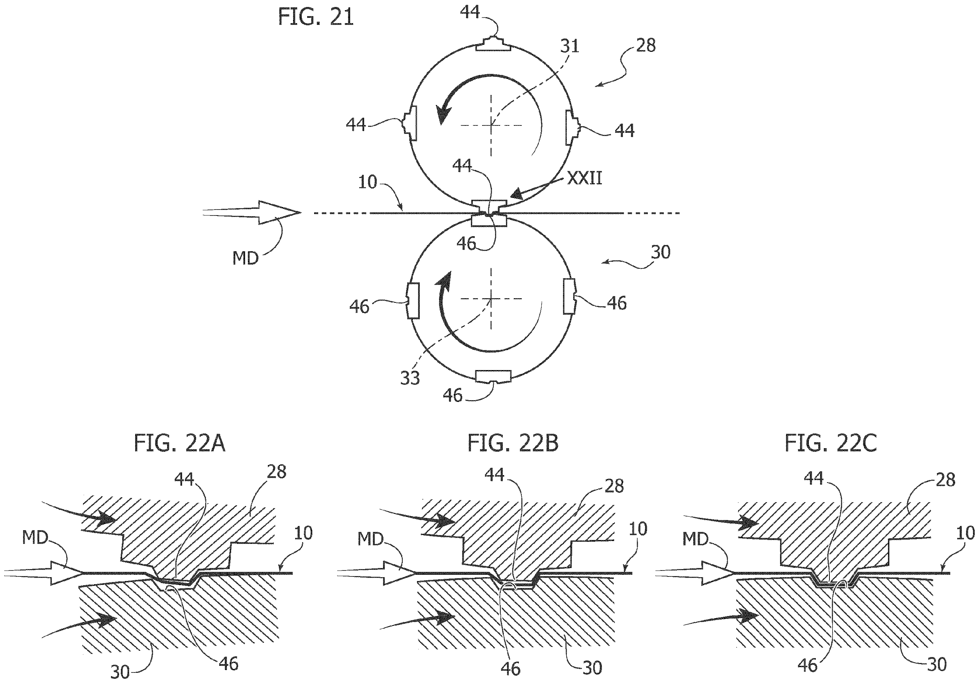

After folding, the method provides for advancing the folded sheet of paper material 10 containing the absorbent sanitary article 16 in the machine direction MD between a first and a second embossing wheel 28, 30 (Figure 21), which rotate around respective fixed rotation axes 31, 33 transverse to the machine direction MD. The first and second embossing wheels 28, 30 can operate at peripheral speeds of up to 250 m/min.

As schematically illustrated in Figures 22A, 22B, and 22C, the first and second embossing wheels 28, 30 are equipped with protrusions and cavities 44, 46 that interpenetrate each other. The mutually facing surfaces of the protrusions 44 and cavities 46 do not come into contact. When the protrusions 44 and cavities 46 interpenetrate, a gap with a thickness greater than 30 µm and less than the thickness of the overlapping flaps of paper material 22, 24, 26 is defined between the respective facing surfaces.

The protrusions 44 and cavities 46 of the embossing wheels 28, 30 locally deform portions of at least two overlapping flaps of paper material 22, 24, 26 to form embossing seams 32, 34 comprising interpenetrating protrusions and recesses 36, 38 which extend through at least one silicone layer 12 and secure at least two overlapping flaps of paper material 22, 24, 26.

Embossing is a mechanical and/or thermal processing technique that involves applying pressure and/or heat to a surface to give it a permanent three-dimensional relief. This technique is often used to create decorative patterns, raised text, or other tactile features on materials such as paper, cardboard, plastic materials, or other substrates.

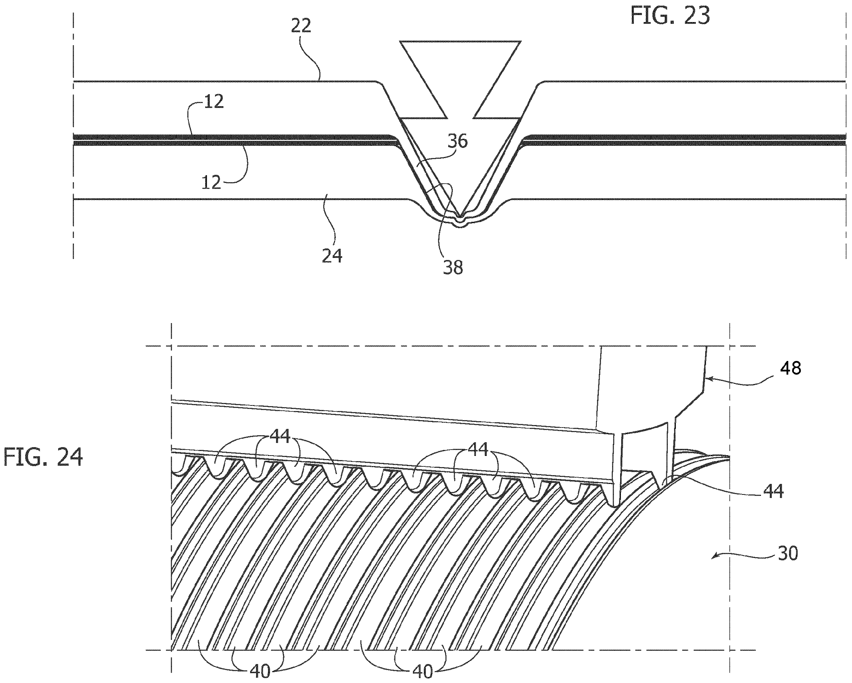

Referring to Figure 23, embossing two overlapping flaps of paper material 22, 24 produces a permanent deformation of the cellulose fibers, creating complementary protrusions and recesses 36, 38 on the opposite surfaces of the two flaps of paper material 22, 24.

The silicone layers 12 are rigid and do not follow the deformation of the paper material. During deformation, the silicone layers 12 locally fracture, forming micro-apertures, and the fibers of one flap of paper material 22 pass through the micro-apertures in the silicone layers 12 and interpenetrate the fibers of the other flap of paper material 24. This forms a mechanical joint that secures the two flaps of paper material 22, 24 together.

In the embossing area, the paper material thins and permanently deforms where the silicone layer 12 fractures, causing permanent plastic deformation. Each section of paper material 10 in the machine direction MD maintains a substantially constant length upstream and downstream of the embossing wheels 28, 30 and does not elongate when passing through the embossing wheels 28, 30.

With reference to Figures 1-6, a first embodiment of the method involves advancing a continuous paper material sheet 10 in the machine direction (MD) and intermittently applying adhesive to the silicone layer 12 to form discrete adhesive areas 14 on the continuous paper material sheet 10, spaced apart from each other in the machine direction (MD).

In this embodiment, the absorbent sanitary articles 16 are oriented with their respective longitudinal axes A transverse to the machine direction MD when applied to the respective discrete adhesive areas 14 of the continuous sheet of paper material 10.

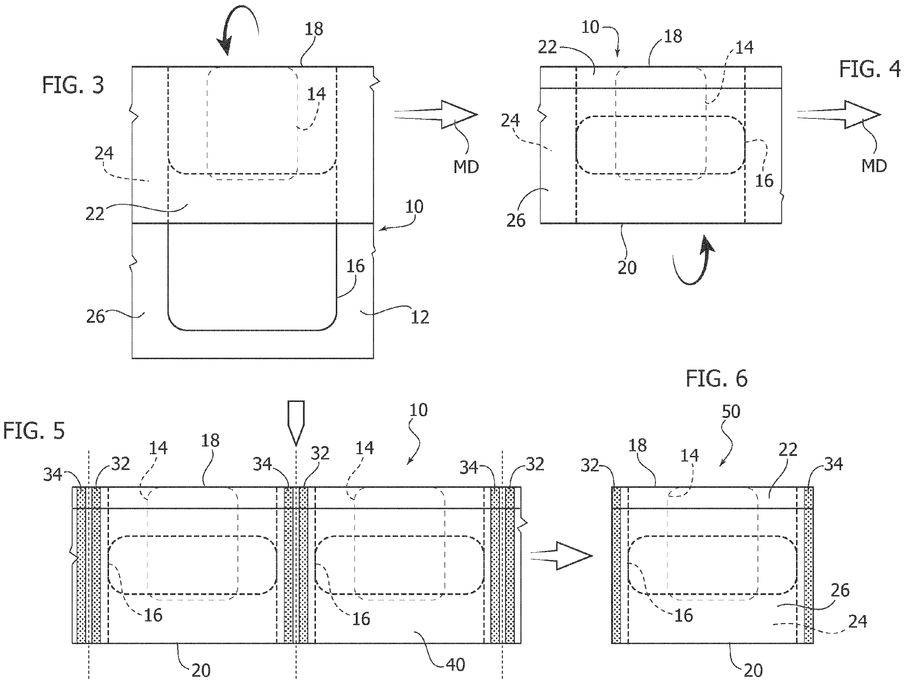

The continuous sheet of paper material 10 and the absorbent sanitary articles 16 are folded along two fold lines 18, 20 parallel to the machine direction MD to form a continuous longitudinal portion 40 with three continuous overlapping flaps of paper material 22, 24, 26 (Figures 3 and 4).

Referring to Figure 5, the tri-folded continuous sheet of paper material 10 containing the tri-folded absorbent sanitary articles 16 is advanced in the machine direction MD between the first and second embossing wheels 28, 30 to form successive pairs of embossing seams 32, 34 transverse to the machine direction MD, located between pairs of adjacent absorbent sanitary articles 16.

Then, as shown in Figures 5 and 6, a transverse cut is made between each pair of embossing seams 32, 34, forming individual packages 50. Each package contains a tri-folded absorbent sanitary article 16 enclosed in a tri-folded paper material wrapper sealed on two opposite sides by embossing seams 32, 34 transverse to the fold lines 18, 20.

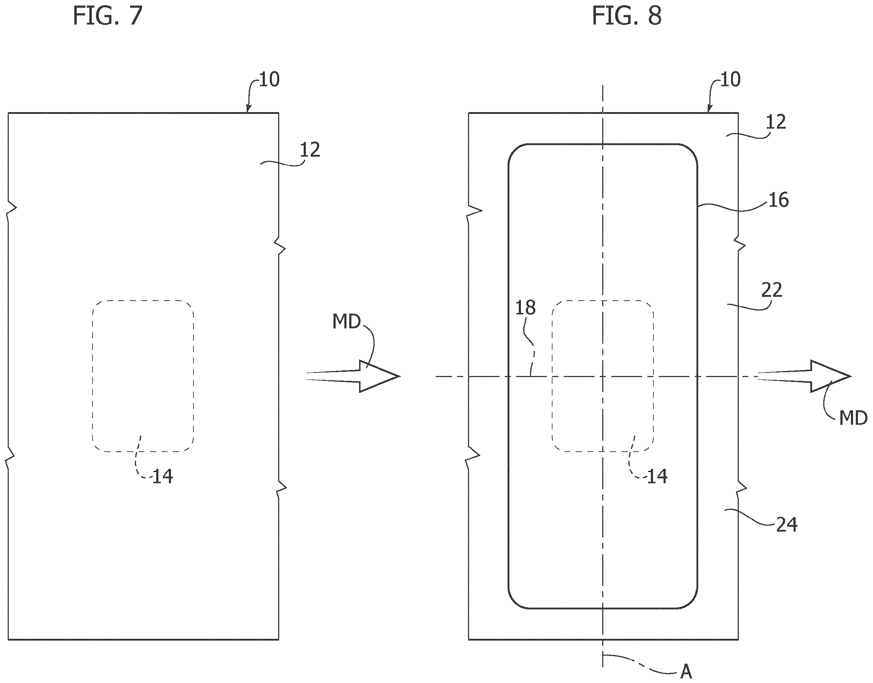

In the embodiment shown in Figures 7-11, similarly to the embodiment of Figures 1-6, the absorbent sanitary articles 16 are oriented with their longitudinal axes A transverse to the machine direction MD when applied to the discrete adhesive areas 14 of the continuous sheet of paper material 10.

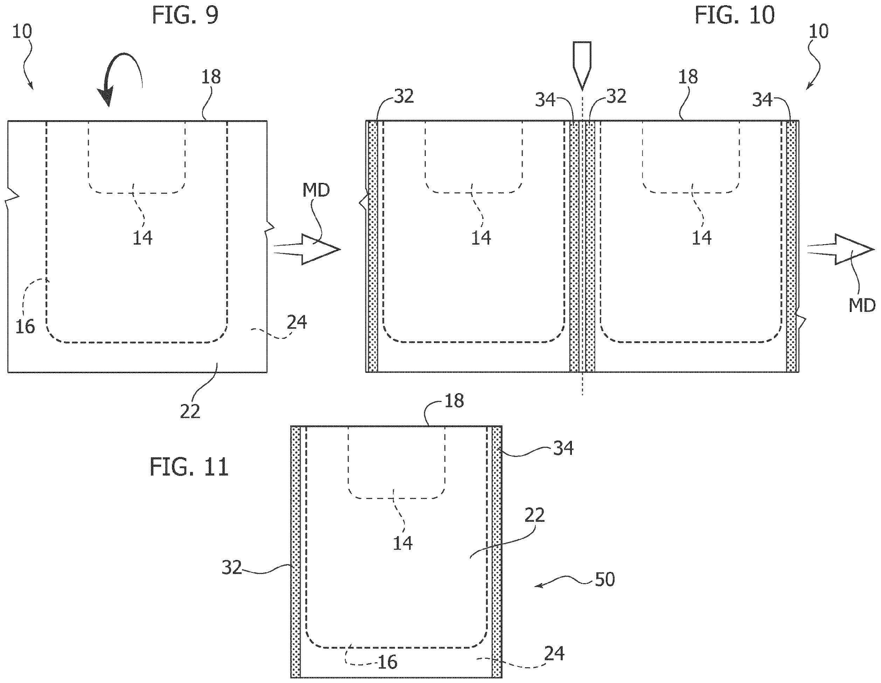

In this second embodiment, the continuous sheet of paper material 10 and the absorbent sanitary articles 16 are folded along a single fold line 18 parallel to the machine direction MD, forming two continuous overlapping flaps of paper material 22, 24 (Figures 8 and 9).

Referring to Figure 10, the bi-folded continuous sheet of paper material 10 containing the bi-folded absorbent sanitary articles 16 is advanced in the machine direction MD between the first and second embossing wheels 28, 30 to form successive pairs of embossing seams 32, 34 transverse to the machine direction MD between adjacent absorbent sanitary articles 16.

Then, as shown in Figures 10 and 11, a transverse cut is made between each pair of embossing seams 32, 34, forming individual packages 50. Each package contains a bi-folded absorbent sanitary article 16 enclosed in a bi-folded paper material wrapper sealed on two opposite sides by embossing seams 32, 34 transverse to the fold line 18.

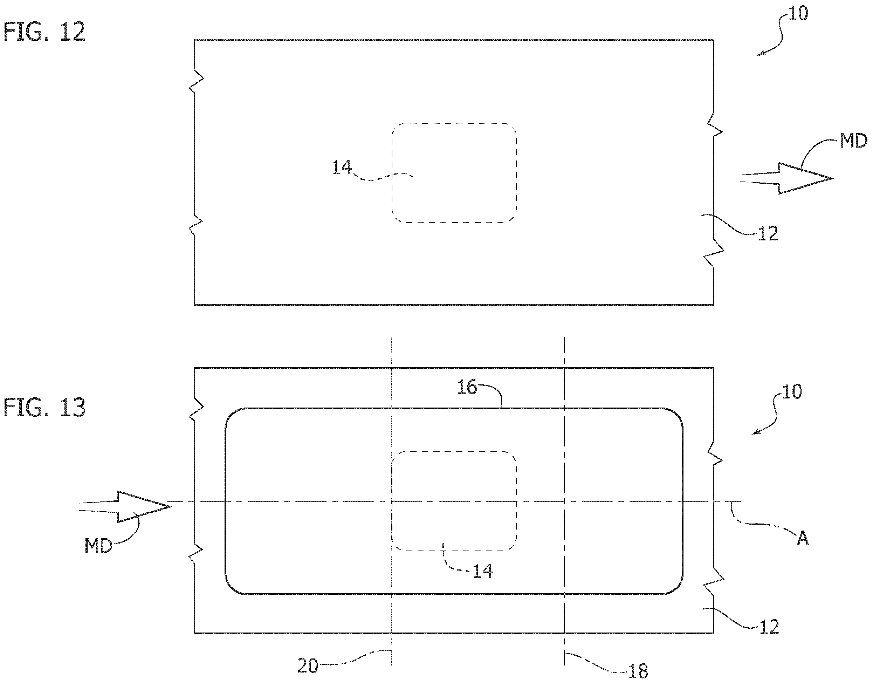

In the embodiment illustrated in Figures 12-16, the absorbent sanitary articles 16 are oriented with their longitudinal axes A parallel to the machine direction MD when applied to the discrete adhesive areas 14 of the continuous sheet of paper material 10.

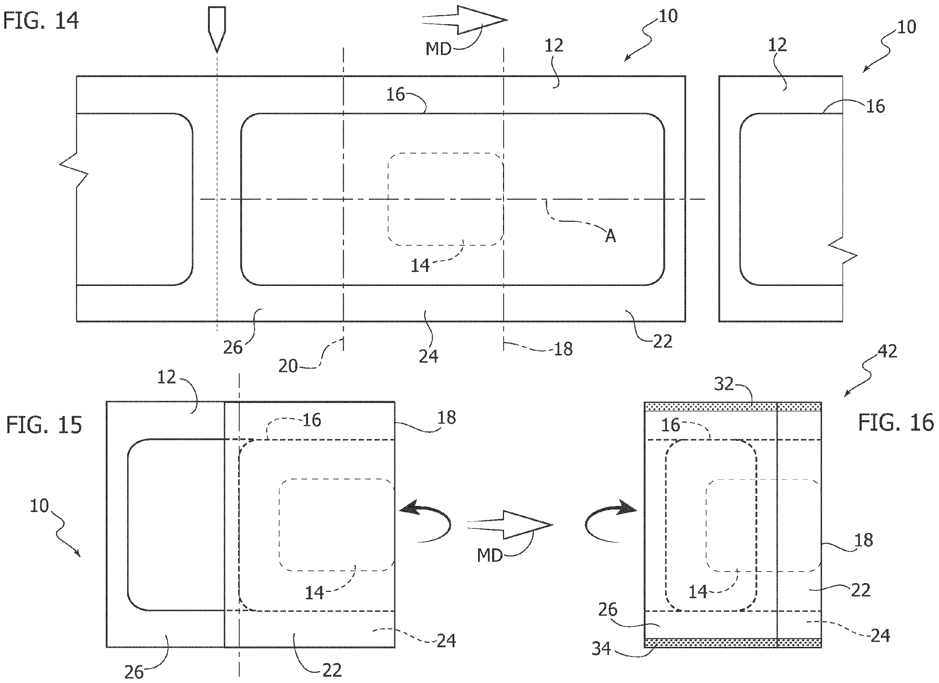

In this embodiment, the method includes cutting the continuous sheet of paper material 10 transversely to the machine direction MD between each pair of adjacent absorbent sanitary articles 16, as shown in Figure 14.

The cut sheet of paper material 10 and the respective absorbent sanitary article 16 are then jointly folded along two fold lines 18, 20 transverse to the machine direction MD to form a package 42 with three overlapping flaps of paper material 22, 24, 26.

Next, the individual packages 42 are advanced in the machine direction MD between the first and second embossing wheels 28, 30. This forms a pair of embossing seams 32, 34 parallel to the machine direction MD along the lateral edges of each package 42.

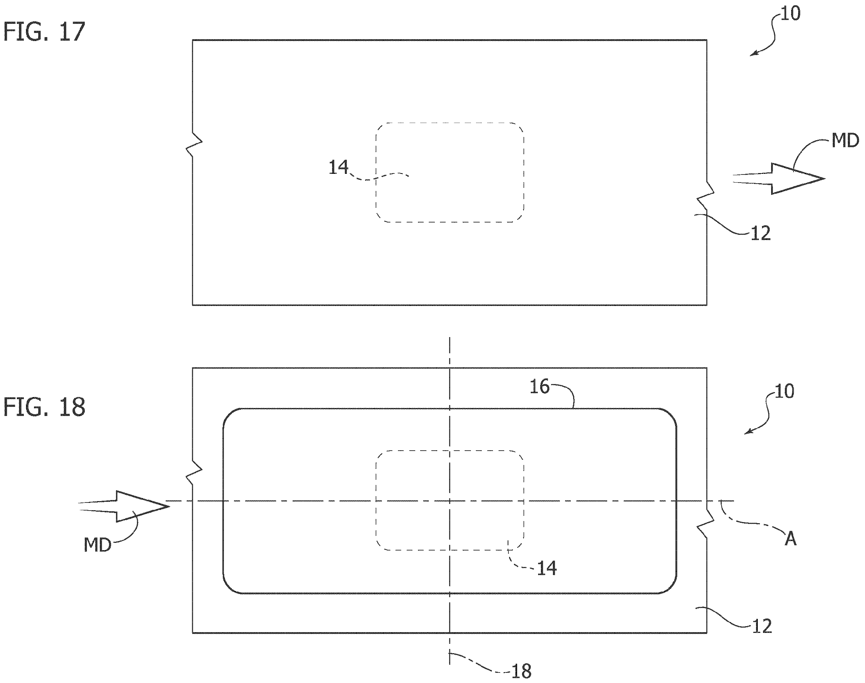

In the embodiment shown in Figures 17-20, similarly to the embodiment of Figures 12-16, the absorbent sanitary articles 16 are oriented with their longitudinal axes A parallel to the machine direction MD when applied to the discrete adhesive areas 14 of the continuous sheet of paper material 10.

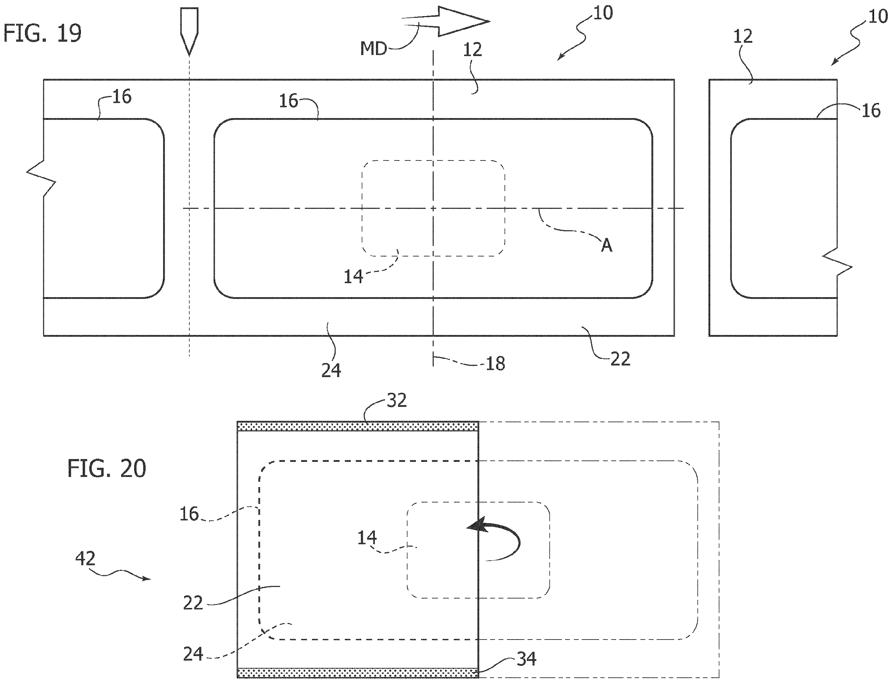

In this embodiment, the method also involves cutting the continuous sheet of paper material 10 transversely to the machine direction MD between each pair of adjacent absorbent sanitary articles 16, as illustrated in Figure 19.

The cut sheet of paper material 10 and the respective absorbent sanitary article 16 are then jointly folded along a single fold line 18 transverse to the machine direction MD to form a package 42 with two overlapping flaps of paper material 22, 24.

As in the embodiment of Figures 12-16, the individual packages 42 are then advanced in the machine direction MD between the first and second embossing wheels 28, 30. This forms a pair of embossing seams 32, 34 parallel to the machine direction MD along the lateral edges of each package 42.

The methods in the embodiments shown in Figures 12-16 and 17-20 produce individual consumer products 50, each comprising a tri-folded or bi-folded absorbent sanitary article 16 enclosed in a tri-folded or bi-folded paper material wrapper sealed on two opposite sides by embossing seams 32, 34 parallel to the fold lines 18, 20.

Referring to Figure 24, in a possible embodiment, the first embossing wheel 28 can be provided with a plurality of protruding elements 48 spaced angularly apart. Each protruding element includes at least one first and one second transverse array of protrusions 44, while the second embossing wheel 30 is provided with annular cavities 46 that are coaxial with each other. The protrusions 44 of each transverse array engage a corresponding annular cavity 46. This embodiment helps to reduce issues related to the alignment between the protrusions 44 and cavities 46 of the embossing wheels 28, 30.

In possible embodiments, the protrusions 44 and the cavities 46 of the first and second embossing wheels 28, 30 can be heated or cooled. The operating temperatures of the protrusions 44 and cavities 46 can range from 0°C to 20°C to facilitate the release of certain materials or from 30°C to 200°C to facilitate the deformation of certain materials. Finally, a range of 0°C to 30°C corresponds to ambient operating temperatures.

In the preceding description, reference has been made to cases where each wrapper contains a respective absorbent sanitary article 16. In possible embodiments, each wrapper may contain two or more absorbent sanitary articles 16.

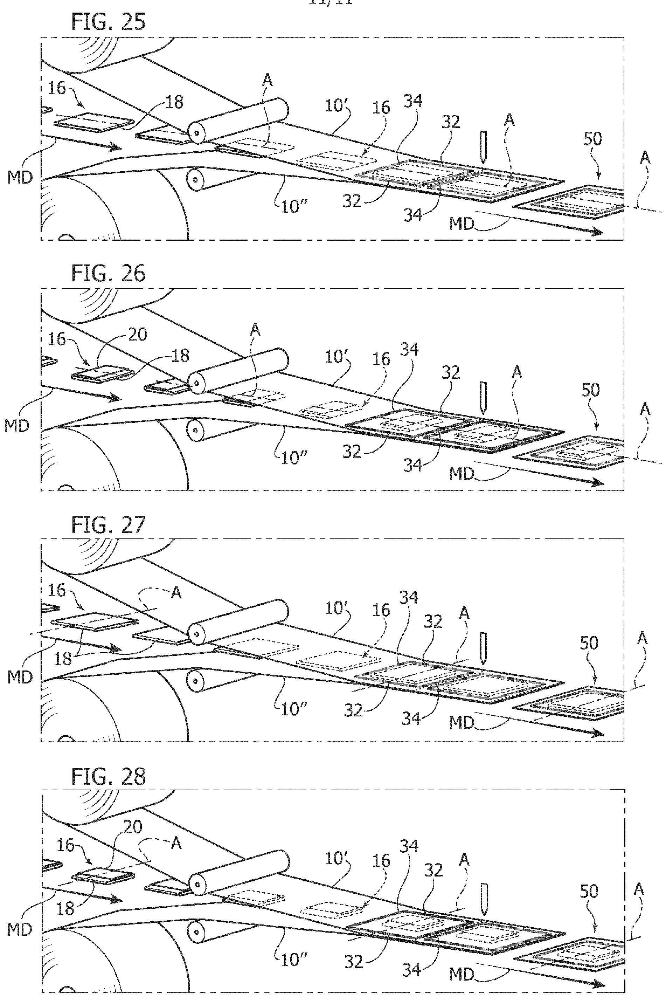

Referring to Figures 25-28, a possible variant involves advancing in the machine direction MD a first and a second sheet of paper material 10', 10", enclosing absorbent sanitary articles 16, either unfolded or folded, between the first and second overlapping sheets of paper material 10', 10".

In Figures 25 and 26, the absorbent sanitary articles 16 can be positioned with their longitudinal axes A parallel to the machine direction MD. In this variant, the absorbent sanitary articles 16 can be folded along a single fold line 18 transverse to the machine direction MD (Figure 25) or along two fold lines 18, 20 transverse to the machine direction MD (Figure 26).

In Figures 27 and 28, the absorbent sanitary articles 16 can be positioned with their longitudinal axes A transverse to the machine direction MD. In this advantageous variant, the absorbent sanitary articles 16 can be folded along a single fold line 18 parallel to the machine direction MD (Figure 27) or along two fold lines 18, 20 parallel to the machine direction MD (Figure 28) .

This variant involves advancing the first and second overlapping sheets of paper material 10', 10" in the machine direction MD, enclosing the absorbent sanitary articles 16 between them, between the first and second embossing wheels 28, 30 (Figure 21), which rotate around respective rotation axes 31, 33 transverse to the machine direction MD, wherein the first and second embossing wheels 28, 30 are equipped with protrusions and cavities 44, 46 that interpenetrate each other.

In this case, the method includes locally deforming portions of the overlapping first and second sheets of paper material 10', 10" between the protrusions and cavities 44, 46 of the embossing wheels 28, 30 to form embossing seams 32, 34 comprising interpenetrating protrusions and recesses 36, 38, which secure at least two sides of the overlapping sheets of paper material 10', 10". In possible embodiments, the embossing seams 32, 34 can be formed parallel to the machine direction MD and transverse to the machine direction MD.

In further possible embodiments, the embossing seams 32, 34 parallel and transverse to the machine direction MD can form quadrangular frames surrounding the respective absorbent sanitary articles 16.

In additional possible embodiments, the embossing seams 32, 34 parallel and transverse to the machine direction MD can be continuous.

In other possible embodiments, the embossing seams 32, 34 parallel and transverse to the machine direction MD can be discontinuous.

This variant may also provide that at least one of the first or second sheets of paper material 10', 10" has a surface covered by a silicone layer.

Furthermore, this variant may provide that both the first and second sheets of paper material 10', 10" have surfaces covered by respective silicone layers.

The variant illustrated in Figures 25-28 implements a method for packaging absorbent sanitary articles, comprising:
- advancing in the machine direction MD a first and second continuous sheet of paper material 10', 10" overlapping each other, at least one of which has a surface covered by a silicone layer 12,
- advancing an array of absorbent sanitary articles 16 between the first and second continuous sheets of paper material 10', 10",
- advancing in the machine direction MD the first and second continuous sheets of paper material 10', 10" and the array of absorbent sanitary articles 16 sandwiched between them, between a first and a second embossing wheel 28, 30 rotating around respective rotation axes 31, 33 transverse to the machine direction MD, wherein said first and second embossing wheels 28, 30 are provided with protrusions and cavities 44, 46 that interpenetrate each other, and
- locally deforming portions of the overlapping first and second continuous sheets of paper material 10', 10" between the protrusions and cavities 44, 46 of the embossing wheels 28, 30 to form on said overlapping first and second continuous sheets of paper material 10', 10" embossing seams 32, 34 comprising interpenetrating protrusions and recesses 36, 38 which extend through at least one silicone layer 12 and secure said overlapping first and second continuous sheets of paper material 10', 10".

Naturally, without prejudice to the principle of the invention, the construction details and the embodiments may be widely varied from what has been described and illustrated without departing from the scope of the invention as defined in the claims that follow.

For example, in additional embodiments, the method for packaging absorbent sanitary articles comprises advancing a sheet of paper material 10 in the machine direction MD.

In this exemplary case, the method also includes applying an absorbent sanitary article 16 to the sheet of paper material 10 so that the absorbent sanitary article 16 is secured to the sheet of paper material 10.

In this exemplary case, the method includes jointly folding the sheet of paper material 10 and the absorbent sanitary article 16 along at least one fold line 18, 20 to form at least two overlapping flaps of paper material 22, 24, 26.

In this exemplary case, the method involves advancing the folded sheet of paper material 10 in the machine direction MD between a first and a second embossing wheel 28, 30 rotating around respective rotation axes 31, 33 transverse to the machine direction MD, wherein the first and second embossing wheels 28, 30 are provided with protrusions and cavities 44, 46 that interpenetrate each other.

In this exemplary case, the method includes locally deforming portions of at least two overlapping flaps of paper material 22, 24, 26 between the protrusions and cavities 44, 46 of the first and second embossing wheels 28, 30 to form embossing seams 32, 34 comprising interpenetrating protrusions and recesses 36, 38, which secure the overlapping flaps of paper material 22, 24, 26 together.

## Claims

1. A method for packaging absorbent sanitary articles, comprising:
- advancing in a machine direction (MD) a sheet of paper material (10) having a surface covered by a silicone layer (12),
- applying and fastening an absorbent sanitary article (16) on said sheet of paper material (10),
- jointly folding said sheet of paper material (10) and said absorbent sanitary article (16) around at least one fold line (18, 20) to form at least two overlapping flaps of paper material (22, 24, 26),
- advancing said sheet of folded paper material (10) in said machine direction (MD) between first and second embossing wheels (28, 30) rotatable around respective rotation axes (31, 33) transverse to said machine direction (MD), wherein said first and second embossing wheels (28, 30) are provided with protrusions and cavities (44, 46) which interpenetrate with each other, and
- locally deforming portions of said at least two overlapping flaps of paper material (22, 24, 26) between said protrusions and cavities (44, 46) of said first and second embossing wheels (28, 30) to form on said at least two overlapping flaps of paper material (22, 24, 26) embossing seams (32, 34) comprising protrusions and recesses (36, 38) interpenetrating with each other which extend through at least one silicone layer (12) and fix to each other said at least two overlapping flaps of paper material (22, 24, 26).

2. The method of claim 1, wherein a gap having a thickness greater than 30 µm and less than the thickness of said overlapping folds of paper material (22) is defined between mutually facing surfaces of said protrusions and cavities (44, 46) interpenetrated with each other (24, 26).

3. The method of any of the preceding claims, wherein said sheet of paper material (10) has a basis weight of between 25 - 50 gsm.

4. The method of any of the preceding claims, wherein said silicone layer (12) has a basis weight between 1 - 3 gsm.

5. The method of any of the preceding claims, wherein a first embossing wheel (28) is provided with protruding elements (48) spaced angularly apart, each of which comprises at least one first and one second transverse array of protrusions (44) and wherein a second embossing wheel (30) is provided with annular cavities (46) coaxial with each other.

6. The method of any of the preceding claims, comprising:
- advancing in said machine direction (MD) a continuous sheet of paper material (10) having a surface covered by a silicone layer (12),
- applying absorbent sanitary articles (16) oriented with respective longitudinal axes (A) transverse to said machine direction (MD) on said continuous sheet of paper material (10) and fastening said absorbent sanitary articles (16) to said continuous sheet of paper material (10),
- jointly folding said continuous sheet of paper material (10) and said absorbent sanitary articles (16) around at least one fold line (18, 20) parallel to said machine direction (MD) to form at least two continuous overlapping flaps of paper material (22, 24, 26),
- advancing in said machine direction (MD) said continuous sheet of folded paper material (10) between said first and second embossing wheels (28, 30), and
- forming on said at least two continuous overlapping flaps of paper material (22, 24, 26) successive pairs of embossing seams (32, 34) transverse to said machine direction (MD) and arranged between pairs of absorbent sanitary articles (16) adjacent to each other.

7. The method of claim 6, comprising:
- jointly folding said continuous sheet of paper material (10) and said absorbent sanitary articles (16) around first and second folding lines (18, 20) parallel to said machine direction (MD) to form a tri-folded continuous sheet of paper material (10) having a continuous longitudinal portion (40) with three continuous overlapping flaps of paper material (22, 24, 26), and
- forming said pairs of embossing seams (32, 34) transverse to said machine direction (MD) on said continuous longitudinal portion (40) with three continuous overlapping flaps of paper material (22, 24, 26) .

8. The method of to any of claims 1 to 5, comprising:
- advancing in said machine direction a continuous sheet of paper material (10) having a surface covered by a silicone layer (12),
- applying absorbent sanitary articles (16) oriented with respective longitudinal axes (A) parallel to said machine direction (MD) on said continuous sheet of paper material (10) and fastening said absorbent sanitary articles (16) to said continuous sheet of paper material (10),
- cutting said continuous sheet of paper material (10) transversally to said machine direction (MD) between each pair of adjacent absorbent sanitary articles (16),
- jointly folding said sheet of cut paper material (10) and the respective absorbent sanitary article (16) around at least one fold line (18) transverse to said machine direction (MD) to form a package (42) with two overlapping flaps of paper material (22, 24), and
- forming embossing seams (32, 34) parallel to said machine direction (MD) along opposite lateral edges of said sheet of folded paper material (10).

9. The method of claim 8, comprising:
- jointly folding said sheet of cut paper material (10) and the respective absorbent sanitary article (16) around first and second folding lines (18, 20) transverse to said machine direction (MD) to form a package (42) having a portion with three overlapping flaps of paper material, and
- forming said embossing seams parallel to said machine direction (MD) on said portion with three overlapping flaps of paper material (22, 24, 26).

10. A machine for manufacturing absorbent sanitary articles enclosed in respective wrappers, comprising a packaging apparatus configured to implement a method according to any of the preceding claims.
